# EUROPEAN PATENT SPECIFICATION

(11) **EP 4 252 177 B1**
(45) Date of publication and mention of the grant of the patent: **26.08.2026**
(21) Application number: 21820559.9
(22) Date of filing: 26.11.2021
(51) Int. Cl.: G06T 3/02, G06T 7/80

(54) **METHOD AND APPARATUS FOR IDENTIFYING IMAGE SHIFT**
VERFAHREN UND VORRICHTUNG ZUR ERKENNUNG EINER BILDVERSCHIEBUNG
PROCÉDÉ ET APPAREIL D'IDENTIFICATION D'UN DÉCALAGE D'IMAGE

(30) Priority: 27.11.2020 GB 202018757
(43) Date of publication of application: 04.10.2023
(73) Proprietor: Elekta Limited, Crawley, West Sussex RH10 9BL (GB)
(72) Inventor: KNOX, Chris, Crawly RH10 9RR (GB); GOODY, James, Crawly RH10 9RR (GB)
(74) Representative: Collins, Emily Jane
(86) International application number: PCT/EP2021/083223
(87) International publication number: WO 2022/112520

(56) References cited:
- US-A1- 2016 023 019
- US-A1- 2018 272 537

## Description

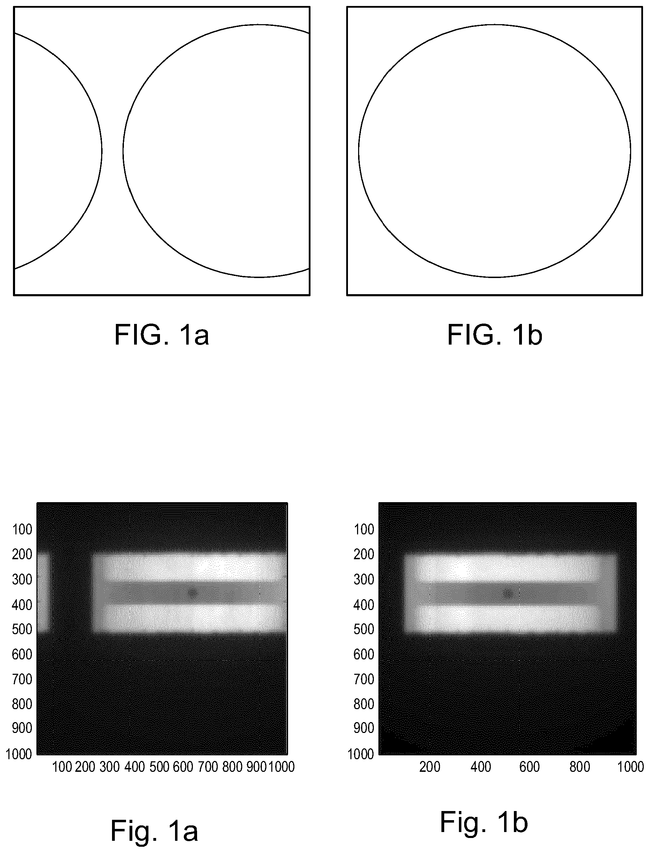

The invention relates to a method and apparatus for identifying image shift. In known image capture or acquisition devices such as the IviewGT (trademark) system available from Elekta AB problems arise with offset of acquired images. Referring to Figs. 1a and 1b, it will be seen that in some instances an image which should appear in a first position as shown in Fig. 1b is in fact offset, for example shifted and wrapped around, as shown in Fig. 1a. Some such shifts can easily be spotted, but small shifts for example with less or no wrap around may be more difficult to identify.

Patent US 2018/272537 A1 (ISHIGAKI TOSHIYUKI [JP] ET AL) (2018-09-27), discloses a robot control device for detecting detection target position from a first image and that corrects detection target position based on a first reference position information stored in a storage medium. Patent US 2016/023019 A1 (FILIBERTI RETO W [CH] ET AL) (2016-01-28) discloses an imaging-based calibration method for imaging-based calibration of radiation treatment couch position compensations, which involves correcting target position by combining beam deviation information with couch position offset information.

The invention will be described by way of example with reference to the figures of which:
Fig. 1a shows an offset acquired image;
Fig. 1b shows the desired image corresponding to Fig. 1a;
Fig. 2 shows a radiotherapy device apparatus according to the present disclosure;
Fig. 3a shows an imagining panel with a reference element according to the present approach;
Fig. 3b shows an imaging panel with a shifted image to be corrected according to the present approach; and
Fig. 4 is a flow diagram illustrating steps preformed according to the present approach.

In overview, a method of identifying an image shift is provided according to claim 1. For example, imaging panel defects can be employed; such defects can appear where the pixels do not behave as expected, for example remaining black or white or displaying another unexpected artefact, or responding differentially compared to other pixels. Where reference markers are employed, these can be identified in a calibration phase or identified by the manufacturer, and are typically filtered out of the image when the image is acquired, but can act as a "finger print" for the panel.

If the acquired image shifts, the position of the reference element will also shift; image processing software can identify the shift and correct the image or prevent the image from being used. As a result, the onus on the user to identify faulty images is significantly reduced, increasing the inherent safety of the imaging device by adjusting, correcting or shifting the image, or discarding it.

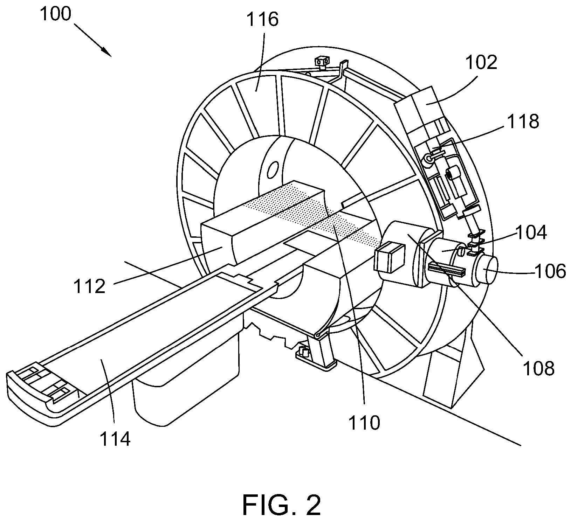

The imaging panels can be used, for example, in a radio therapy device. Fig. 2 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in figure 2 is in accordance with the present disclosure and is suitable for use with the disclosed systems and apparatuses. While the device in figure 2 is an MR-Linac, the implementations of the present disclosure may be any radiotherapy device, for example a Linac device.

The device comprises both MR imaging apparatus 112 and radiotherapy (RT) apparatus which may comprise a Linac device. The MR imaging apparatus is shown in cross-section in the diagram. In operation, the MR scanner produces MR images of the patient, and the Linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-Linac device 100 depicted in figure 2 comprises a source of radiofrequency waves 102, a waveguide 104, a source of electrons 106, a source of radiation, a collimator 108 such as a multi-leaf collimator configured to collimate and shape the beam, MR imaging apparatus 112, and a patient support surface 114. In use the device would also comprise a housing (not shown) which, together with the ring-shaped gantry defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation 106 and a radiation detector (not shown). Typically, the radiation detector is positioned diametrically opposed to the radiation source 106. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source 106 may comprise a beam generation system. For a Linac, the beam generation system may comprise a source of RF energy 102, an electron gun 106, and a waveguide 104. The radiation source is attached to the rotatable gantry 116 so as to rotate with the gantry 116. In this way, the radiation source 106 is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the delivery of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the Linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path may be controlled by a suitable arrangement of steering magnets, or steering coils, which surround the waveguide 104. The arrangement of steering magnets may comprise, for example, two sets of quadrupole magnets.

Once the electrons have been accelerated, they may pass, in some Linac configurations, into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The waveguide includes a source of radiation configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation 106 is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the Linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table.

The radiotherapy apparatus / device depicted in figure 2 also comprises MR imaging apparatus 112. The MR imaging apparatus 112 is configured to obtain images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus; an RT apparatus processor, which controls the operation of the RT apparatus; and a subject support surface processor which controls the operation and actuation of the subject support surface. The controller is communicatively coupled to a memory, i.e. a computer readable medium.

The Linac device also comprises several other components and systems as will be understood by the skilled person. For example, in order to ensure the Linac does not leak radiation, appropriate shielding is also provided.

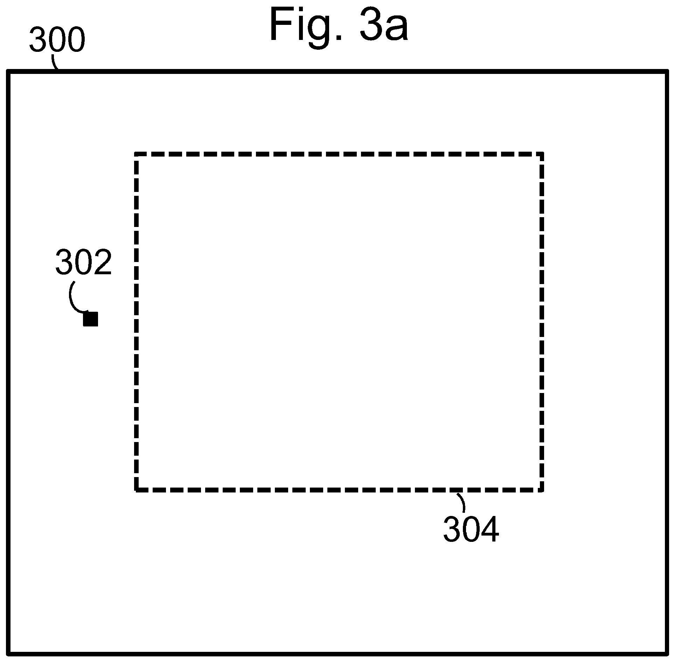

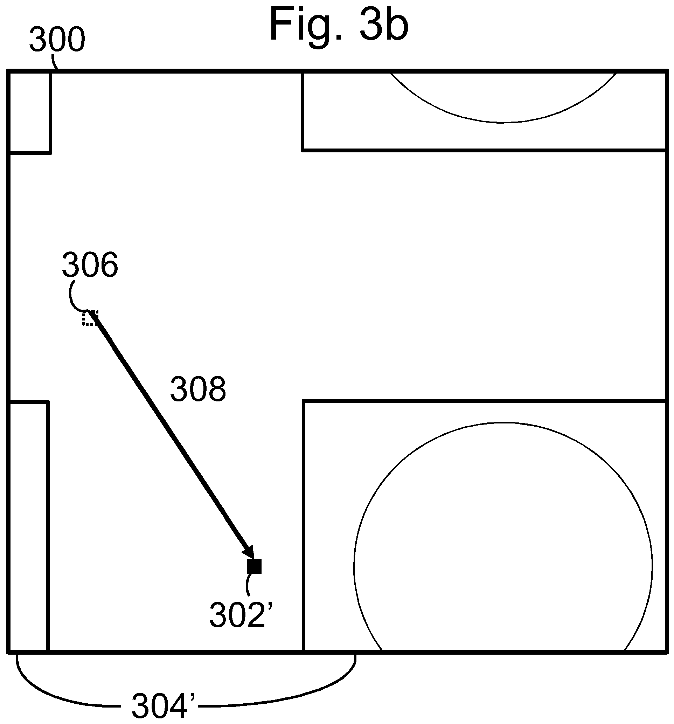

As discussed above, image capture issues can arise in known imaging systems for example of the type discussed above in MR-Linacs as a result of image shift, offset or wraparound. According to Figs. 3a and 3b, this can be overcome by use of a reference marker. Fig. 3a shows an imaging panel 300 including, as shown at 304, the correct position of an image, in dotted lines. Additionally, the imaging panel includes a reference element or marker such as a faulty pixel 302. As discussed in more detail below the faulty pixel can be, for example, faulty, failed, inactive, deficient or otherwise performing differentially from other pixels. Of course, there may be more than one such pixel. Indeed, where multiple faulty pixel locations are known this can assist in identifying, determining the location of and using the information thus derivable from the faulty pixel. An effect of using multiple faulty pixel locations is that noise associated with a single faulty pixel, which may result in varying intensity values, may mean that it may not always be identifiable; multiple pixels mitigate this. Yet further, where multiple reference positions are known, additional information is available in case of readout error which could otherwise lead to different columns of a tiled imager being read in the wrong order. For example, a column could be identified based on intensity value of the reference marker/pixel, or even based on its "y" position, permitting correct image reconstruction.

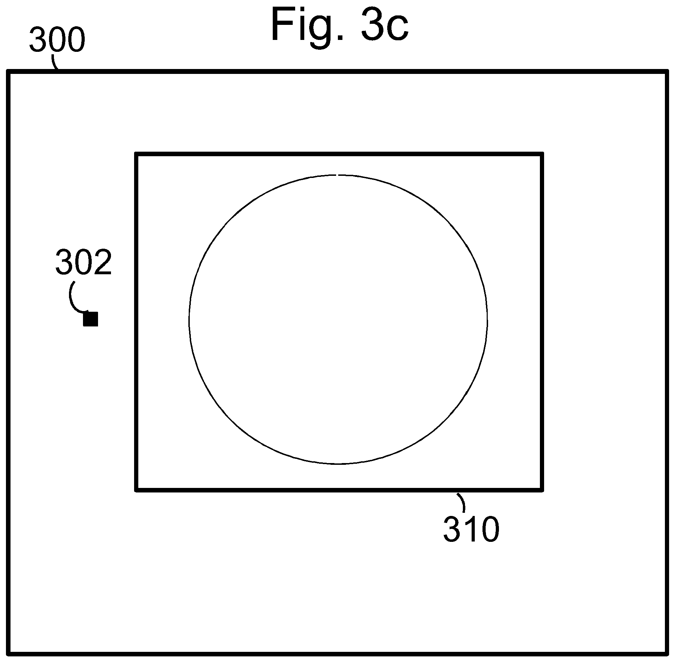

When the image is captured, in instances where there is image shift, an arrangement such as that shown in Fig. 3b may arise. In particular it can be seen that the image is shifted and wrapped around in both the X and Y direction as shown at 304'. Of course, any form of offset, transposition, incorrect reordering, shift or wraparound may occur and the version shown in Fig. 3b is an extreme one for purposes of explanation. The predetermined expected position of the faulty pixel is shown at 306 but it can be seen that it is shifted in a captured image in the same manner as the remainder of the image to a position 302'. The shift can be represented by a shift vector 308. As a result, the image can be rectified or corrected as shown in Fig. 3c where the image 310 is repositioned correctly to correspond to the desired image shown in Fig. 3a, but shifting it back by the same operation applied to the faulty pixel shown in its correct position at 302 in Fig. 3c. This can be achieved for example by effectively applying a transformation comprising translation according to the inverse of the shift vector 308 in Fig. 3b.

**It** will be noted that the shifted image can be detected in alternative manners, for example by modifying imaging hardware to put reference markers in the image such that rather than having a faulty pixel 302, a reference marker is provided. Once again, once the image is captured, any shift in position of the reference marker can be presented by a shift vector or other transformation representation, and the shift can be rectified by reversing the transformation.

In one embodiment the reference marker can be a pixel or other marker deliberately placed at the time of, or after manufacture of the imaging detector. A reference value, for example an intensity value as a percentage of maximum output, associated with the pixel can be set to permit it to be identified consistently. As a result, the pixel can be identified from one image to the next allowing image shift to be accurately and consistently determined.

In an embodiment specific shapes or patterns of pixels or markers could be inserted to allow them to be differentiated from each other. Yet further, or alternatively, the marker can be provided by predefining the location value of one or more pixels to provide an encoded geometric location of the pixel or pixels. A coded pattern can, additionally, reduce the risk of areas being transposed by failures of the device, for example where the device is a 'tiled' device. Imaging devices based for example on amorphous silicon sensors can be singular or tiled, but in either case the readout and processing electronics are often modular and can encounter failures in addressing potential causes of incorrect imaging, for example distinguishing between a transposition or a wrap - again knowledge of the relative position/shape/coding of multiple reference markers can provide additional reconstruction information.

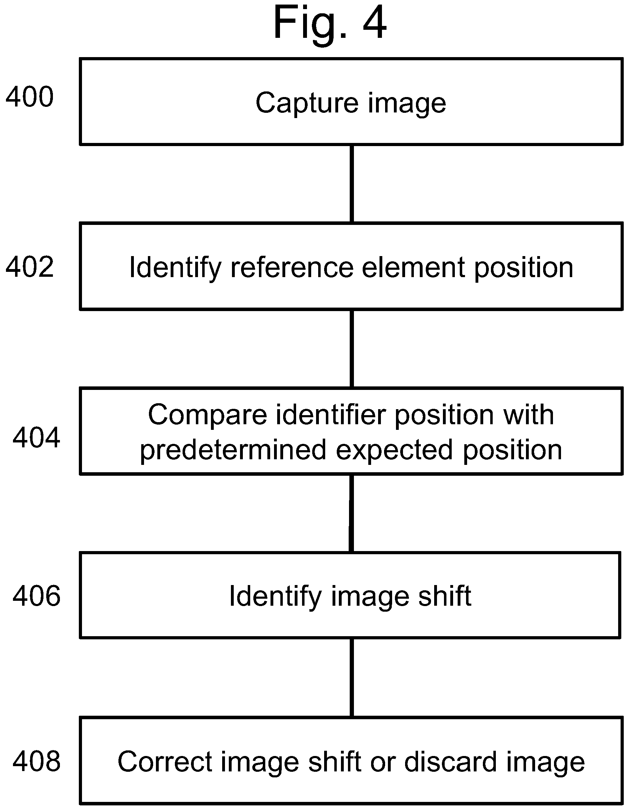

Operation of the approach is shown in Fig. 4. At step 400 an image is captured and a reference element position is identified such a bad pixel position. This is compared, at step 404, with the predetermined expected position. For example, where the bad pixel position is known on the image capture array such as a bad CCD pixel, or where a reference marker such as a known alternatively functioning pixel is provided, this position will be known from a calibration phase or from manufacturer information and can be compared with the location of the known position of the captured image. At step 406, the image shift is identified and at step 408 the image shift is managed appropriately, for example by correcting it by applying an image shift vector inversion or other transformation, discarding the image or other steps.

**It** will be noted that the approaches described herein can be implemented in any appropriate manner. For example, where images are captured using a CCD panel, then in a calibration step the bad pixel location can be identified. This can be a standard calibration process using data that is currently used to edit out faulty pixels in the acquired image or can be an additional calibration stage specifically to identify the location for subsequent steps as discussed above. The image capture panel can be of any appropriate kind for example the Iview or XVI panels such as those used in the Elekta Unity product. Alternatively, and as discussed above, the reference marker can be deliberately introduced by having an inactive or otherwise differentiated pixel in the capture array, the position of which is known from manufacture information or calibration and used in the image shift detection approach as discussed in more detail above.

As discussed above the imaging panel can be used in any appropriate apparatus for example a radiotherapy device. Any type of appropriate imaging hardware can be used, for example any type of readout device where a known reference marker or an encoded pattern is used to validate (and reject) or correct the image data. **It** will further be seen that by harnessing the information provided by faulty pixel location the lifetime of the device can be prolonged rather than simply discarding the device as faulty.

## Claims

1. A computer-implemented method of identifying image wrap-around comprising identifying (402) a position of a reference element in a captured image from an image capture device, comparing (404) the identified position with a predetermined expected position, and identifying (406) image wrap-around if the compared positions do not match; in which the reference element (302) comprises a differentially operating pixel of the image capture device, which operates differently from other pixels.

2. A method as claimed in claim 1 in which the differentially operating pixel comprises a faulty pixel.

3. A method as claimed in claim 1 in which the reference element comprises a reference marker.

4. A method as claimed in claim 3 in which the reference marker has a known intensity.

5. A method as claimed in any preceding claim in which the reference element comprises multiple pixels having predetermined expected positions.

6. A method as claimed in claim 5 further comprising identifying the captured image as disordered if the pixels are not in their predetermined expected positions, and reordering the captured image such that the pixels occupy predetermined expected positions.

7. A method as claimed in any preceding claim further comprising the step of discarding (408) the captured image if the compared positions do not match.

8. A method as claimed in any of claims 1 to 7 further comprising the step of correcting (408) the captured image if the compared positions do not match.

9. A method as claimed in claim 8 comprising identifying a shift vector corresponding to the identified image wrap-around and correcting the image by applying the inverse shift vector to the captured image.

10. A method as claimed in any preceding claim in which the predetermined expected position is determined in a calibration phase or is provided by an imaging panel manufacturer.

11. A computer readable medium comprising instructions, which, when executed by a processor, perform the method of any of claims 1 to 10.

12. An apparatus comprising an image capture device and a processor arranged to operate according to the instructions contained in the computer readable medium of claim 11.

13. A radiotherapy system comprising the apparatus of claim 12 .

## Patentansprüche

1. Computerimplementiertes Verfahren zum Identifizieren einer Bildumwicklung, umfassend Identifizieren (402) einer Position eines Referenzelements in einem aufgenommenen Bild von einer Bilderfassungsvorrichtung, Vergleichen (404) der identifizierten Position mit einer vorbestimmten erwarteten Position und Identifizieren (406) einer Bildumwicklung, falls die verglichenen Positionen nicht übereinstimmen; wobei das Referenzelement (302) ein differentiell arbeitendes Pixel der Bilderfassungsvorrichtung umfasst, das anders als andere Pixel arbeitet.

2. Verfahren nach Anspruch 1, wobei das differentiell arbeitende Pixel ein fehlerhaftes Pixel umfasst.

3. Verfahren nach Anspruch 1, wobei das Referenzelement eine Referenzmarkierung umfasst.

4. Verfahren nach Anspruch 3, bei dem der Referenzmarker eine bekannte Intensität aufweist.

5. Verfahren nach einem der vorhergehenden Ansprüche, wobei das Referenzelement mehrere Pixel mit vorbestimmten erwarteten Positionen umfasst.

6. Verfahren nach Anspruch 5, ferner umfassend Identifizieren des aufgenommenen Bildes als ungeordnet, wenn sich die Pixel nicht in ihren vorbestimmten erwarteten Positionen befinden, und Neuordnen des aufgenommenen Bildes, so dass die Pixel vorbestimmte erwartete Positionen einnehmen.

7. Verfahren nach einem der vorhergehenden Ansprüche, das ferner den Schritt des Verwerfens (408) des aufgenommenen Bildes umfasst, wenn die verglichenen Positionen nicht übereinstimmen.

8. Verfahren nach einem der Ansprüche 1 bis 7, das ferner den Schritt des Korrigierens (408) des aufgenommenen Bildes umfasst, wenn die verglichenen Positionen nicht übereinstimmen.

9. Verfahren nach Anspruch 8, umfassend Identifizieren eines Verschiebungsvektors, der dem identifizierten Bildumschlag entspricht, und Korrigieren des Bildes durch Anwenden des inversen Verschiebungsvektors auf das erfasste Bild.

10. Verfahren nach einem der vorhergehenden Ansprüche, bei dem die vorbestimmte erwartete Position in einer Kalibrierungsphase bestimmt wird oder von einem Bildgebungsplattenhersteller bereitgestellt wird.

11. Ein computerlesbares Medium, das Anweisungen umfasst, die bei Ausführung durch einen Prozessor das Verfahren gemäß einem der Ansprüche 1 bis 10 durchführt.

12. Vorrichtung, die eine Bilderfassungsvorrichtung und einen Prozessor umfasst, die dazu ausgelegt sind, gemäß den Anweisungen zu arbeiten, die in dem computerlesbaren Medium nach Anspruch 11 enthalten sind.

13. Strahlentherapiesystem, das die Vorrichtung nach Anspruch 12 umfasst.

## Revendications

1. Procédé mis en œuvre par ordinateur pour identifier un enroulement d'image, le procédé comprenant d'identifier (402) la position d'un élément de référence dans une image capturée au moyen d'un dispositif de capture d'image, de comparer (404) la position identifiée avec une position attendue prédéterminée, et d'identifier (406) un enroulement d'image si les positions comparées ne correspondent pas ; où l'élément de référence (302) comprend un pixel fonctionnant de manière différentielle du dispositif de capture d'image, qui fonctionne différemment des autres pixels.

2. Procédé selon la revendication 1, dans lequel le pixel fonctionnant de manière différentielle comprend un pixel défectueux.

3. Procédé selon la revendication 1, dans lequel l'élément de référence comprend un marqueur de référence.

4. Procédé selon la revendication 3, dans lequel le marqueur de référence a une intensité connue.

5. Procédé selon l'une quelconque des revendications précédentes, dans lequel l'élément de référence comprend de multiples pixels ayant des positions attendues prédéterminées.

6. Procédé selon la revendication 5, comprenant en outre d'identifier l'image capturée comme étant désordonnée si les pixels ne sont pas dans leurs positions attendues prédéterminées, et de réorganiser l'image capturée de telle sorte que les pixels occupent des positions attendues prédéterminées.

7. Procédé selon l'une quelconque des revendications précédentes, comprenant en outre l'étape comprenant de rejeter (408) l'image capturée si les positions comparées ne correspondent pas.

8. Procédé selon l'une quelconque des revendications 1 à 7, comprenant en outre l'étape comprenant de corriger (408) l'image capturée si les positions comparées ne correspondent pas.

9. Procédé selon la revendication 8, comprenant d'identifier un vecteur de décalage correspondant à l'enroulement d'image identifié et de corriger l'image en appliquant le vecteur de décalage inverse à l'image capturée.

10. Procédé selon l'une quelconque des revendications précédentes, dans lequel la position attendue prédéterminée est déterminée dans une phase d'étalonnage ou est fournie par un fabricant de panneaux de formation d'image.

11. Support lisible par ordinateur comprenant des instructions qui, lorsqu'elles sont exécutées par un processeur, exécutent le procédé selon l'une quelconque des revendications 1 à 10.

12. Appareil comprenant un dispositif de capture d'image et un processeur conçu pour fonctionner conformément aux instructions contenues dans le support lisible par ordinateur selon la revendication 11.

13. Système de radiothérapie comprenant l'appareil selon la revendication 12.
